# EUROPÄISCHE PATENTSCHRIFT

(11) **EP 0 356 695 B1**
(45) Veröffentlichungstag und Bekanntmachung des Hinweises auf die Patenterteilung: **04.01.1995**
(21) Anmeldenummer: 89113504.8
(22) Anmeldetag: 22.07.1989
(51) Int. Cl.: C12N 15/10, C12N 15/41

(54) **in vitro Synthese einer infektiösen RNA**
In vitro synthesis of an infectious RNA
Synthèse in vitro d'une ARN infectieuse

(30) Priorität: 25.07.1988 DE 3825189; 24.06.1989 DE 3920753
(43) Veröffentlichungstag der Anmeldung: 07.03.1990
(73) Patentinhaber: BOEHRINGER INGELHEIM INTERNATIONAL GmbH, 55218 Ingelheim am Rhein (DE)
(72) Erfinder: Duechler, Markus, A-1040 Wien (AT); Skern, Timothy, Dr., A-1160 Wien (AT); Blaas, Dieter, Dr., A-1090 Wien (AT); Berger, Berthold, Dipl.-Ing., A-1130 Wien (AT); Sommergruber, Wolfgang, Dr., A-1130 Wien (AT); Kuechler, Ernst, Prof. Dr., A-1190 Wien (AT)

(56) Entgegenhaltungen:
- PROC. NATL. ACAD. SCI. USA, Band 83, April 1986, Seiten 2330-2334; S. VAN DER WERF et al.: "Synthesis of infectious poliovirus RNA by purified T7 RNA polymerase"
- NUCLEIC ACIDS RESEARCH, Band 13, Nr. 6, 1985, Seiten 2111-2116; T. SKERN et al.: "Human rhinovirus 2: complete nucleotide sequence and proteolytic processing signals in the capsid protein region"
- JOURNAL OF VIROLOGY, Band 56, Nr. 2, November 1985, Seiten 628-632, American Society for Microbiology; S. MIZUTANI et al.: "In vitro synthesis of an infectious RNA from cDNA clones of human rhinovirus type 14"
- CELL BIOLOGY OF VIRUS ENTRY, REPLICATION, AND PATHOGENESIS, Band 90, 25.Februar - 5. März 1988, Seiten 75-83, A.R. Liss Inc; R.J. COLONNO et al.:"Interaction of cellular receptors with the canyon structure of human rhinoviruses"
- VIROLOGY, Band 168, 1989, Seiten 159-161, Academic Press Inc.; D. BLAAS et al.:"Human Rhinovirus serotype 2: In Vitro synthesis of an infectious RNA"
- PROTEINS: STRUCTURE, FUNCTION, AND GENETICS, Band 2, Nr. 4, 1987, Seiten 263-272, A.R. Liss Inc.; D. BLAAS et al.: "Comparison of the three-dimensional structure of two human rhinoviruses (HRV2 and HRV14)"

## Beschreibung

Gegenstand der vorliegenden Erfindung ist die in vitro Synthese einer infektiösen RNA von HRV2 sowie sogenannte "full size" Klone von HRV2.

Humane Rhinoviren sind die Hauptverursacher von Schnupfen (1). Obwohl mehr als einhundert antigenisch unterschiedliche Serotypen existieren, binden mit Ausnahme von 10 Serotypen alle bisher getesteten an denselben Rezeptor (2). Jüngste Fortschritte der Rhinovirusforschung, wie die Bestimmung der Nukleotidsequenz von 4 Serotypen und die Bestimmung der Kristallstruktur von HRV14, haben zwar zu einem besseren Verständnis der Vielfalt der Serotypen geführt (3, 4, 5, 6, 7, 8), doch ist es nach wie vor noch nicht möglich, eine bleibende oder zumindest langdauernde Immunität gegen rhinovirale Infektionen aufzubauen. Nach erfolgter Infektion lassen sich zwar Antikörper gegen den betreffenden Stamm nachweisen, doch gewähren diese keinen Schutz vor anderen Rhinovirusstämmen. Eine vorbeugende Impfung, wie sie beispielsweise möglich ist bei Polio- oder Influenzaviren ist zumindest z.Zt. ausgeschlossen.

Um eine wirksame Therapiemöglichkeit gegen rhinovirale Infektionen zu entwickeln, ist es daher unerläßlich, das in der Zelle ablaufende virale Geschehen zu analysieren.

Voraussetzung hierfür ist ein Testsystem mit dem sich beispielsweise Veränderungen auf ihre Auswirkungen auf das virale Geschehen untersuchen lassen.

Eine Aufgabe der vorliegenden Erfindung bestand darin, ein Testsystem bereitzustellen, mit dem sich beliebige Mutationen, gezielt auf der DNA-Ebene erzeugt, untersuchen lassen.

Erfindungsgemäß gelöst wurde die Aufgabe durch die Synthese einer cDNA-Kopie von HRV2, die es ermöglicht in vitro infektiöse RNA zu transkribieren. Mit dieser synthetischen cDNA ist es überraschenderweise möglich, das virale Geschehen für HRV2 in vitro zu imitieren.

Die Auswirkungen jedweder Mutationen auf der DNA-Ebene wie beispielsweise Deletionen, Insertionen und Austausch von Basenpaaren lassen sich damit direkt und quantitativ meßbar untersuchen.

Erkenntisse, die sich mit Hilfe dieses Testsystems ermitteln lassen, sind aufgrund der weit größeren Homologie des HRV2 zu den bisher sequenzierten Rhinoviren als HRV14, eher zu verallgemeinern, als die mit dem von Mizutani et al. (9) erhältlichen "full size clon" von HRV14. Obwohl dieser Klon 21 Nukleotide mehr als die "native" DNA enthielt konnte mit ihm infektiöse RNA erhalten werden. Racaniello et al. (18) konnten zeigen, daß eine vollständige cDNA Kopie von Poliovirus infektiöses Virus produzieren kann.

Von keinem der zur sogenannten "minor receptor group" gehörenden Rhinoviren ist bisher die Synthese eines cDNA Klons beschrieben worden, von der eine infektiöse RNA transkribiert werden konnte.

cDNA Klone von HRV2 sind zwar beschrieben worden (6), doch ist die durchschnittliche Länge von 0,5 kb zur Konstruktion einer vollständigen cDNA unvorteilhaft.

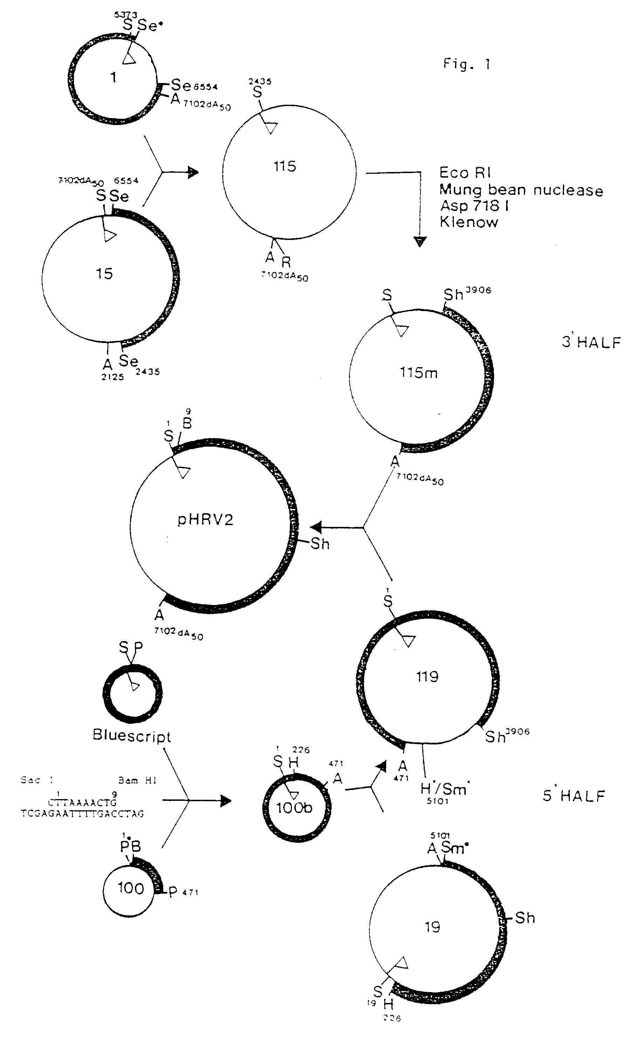

Zur Lösung der erfindungsgemäßen Aufgabe wurde daher wie folgt verfahren:
Die Synthese des ersten Stranges von HRV2 RNA (5 µg) wurde wie beschrieben durchgeführt (10). Diese cDNA wurde dann mit RNase H und DNA Polymerase 1 doppelsträngig gemacht, wobei die Reagenzien von Amersham verwendet wurden. T4 DNA Polymerase wurde verwendet, um Überhänge abzubauen. Nach Behandlung mit Eco RI Methylase wurden Eco RI Linker angefügt und die cDNA wurde mit Eco RI verdaut. Moleküle, die länger als 4 kb waren, wurden von einem 1 % Agarosegel isoliert und in die Eco RI Stelle des Plasmids Bluescript (Stratagene, San Diego, California) ligiert. Die Rekombinanten werden sequenziert, um festzustellen, ob sämtliche Regionen der HRV2-Sequenz abgedeckt sind. Die Bereiche, die nicht abgedeckt sind, werden entweder durch chemische Synthese oder durch Wiederholung der oben beschriebenen Schritte bereitgestellt. Diese Klone werden dann zu einem vollständigen cDNA Molekül zusammengesetzt. Am 3′-Ende der vollständigen cDNA von HRV2 wird ein zur Transkription geeigneter RNA-Polymerase Promotor, beispielsweise der T7 RNA Polymerase Promotor oder der SP6 RNA Polymerase Promotor angefügt. Im vorliegenden Fall wurden cDNA Rekombinanten einer Größe bis zu 6 kb erhalten (Fig. 1). Da keiner der Klone jedoch das vollständige 5′ Ende enthielt, wurde ein Klon, der die Nukleotide 1 bis 471 enthielt und schon früher beschrieben worden war (p100, (6)) verwendet, um diese Region abzudecken.

4 cDNA Klone: p100 (mit dem größeren Pst I Fragment für die Nukleotide 1 - 471), p19 (19 - 5101), p15 (2125 - 7102dA50) und p1 (5373 - 7102dA50) wurden verwendet, um ein vollständiges cDNA Molekül zusammenzusetzen, das von der T7 RNA Polymerase transkribiert werden konnte. Die in Fig. 1 gezeigte Strategie wurde verwendet, um zwei Segmente entsprechend den 5′ und 3′ Teilen des Moleküls zusammenzusetzen. Für den 5′ Teil wurde das 0,6 kb Bam HI/Pst I Fragment von Klon 100 in die Sac I/Pst I Stelle des Plasmids Bluescript eingebaut. Dieses Plasmid enthält den T7 RNA Polymerase Promotor neben dem Polylinker. Ein synthetisches, doppelsträngiges Oligonukleotid, das die Nukleotide 1 bis 9 von HRV2 und die korrekten überhängenden Enden enthielt wurde verwendet, um die Ligation in die Sac I und Bam HI Stellen zu ermöglichen. Die Bluescript Sac I Stelle wurde ausgewählt, weil sie dem T7 RNA Polymerase Promotor am nächsten liegt. Das daraus resultierende Plasmid wurde p100b genannt und enthält die Nukleotide 1 - 471. Es wurde mit Hpa I am Nukleotid 226 von HRV2 geöffnet. Das isolierte 4,9 kb Hpa I/Sma I Fragment des Klons 19 wurde eingebaut um zu Klon 119 zu kommen Nukleotide 1 bis 5101). Diese Konstruktion führt zwar zu einer weiteren Insertion der Nukleotide 226 bis 471 der HRV2 Sequenz zwischen die Hpa°/Sma°I und AspI Restriktionsstelle, doch wird sie bei den nachfolgenden Klonierungsschritten wieder entfernt. (Fig. 1).

Ein Plasmid, das den 3′ Teil enthält, wurde anschließend wie folgt zusammengebaut: Die Klone 1 und 15 wurden miteinander verbunden, indem das 1,2 kb Spe I/Spe I Fragment des Klons 1 (die Spe I Stellen sind im Polylinker und an der Position 6554 im HRV2) durch das 4,1 kb Spe I/Spe I Fragment von Klon 15 ersetzt wurde; es resultierte Klon 115 (Nukleotide 2435 bis 7102dA50). Es war nicht möglich, Klon 15 direkt zu verwenden, da der polydA-Trakt der Sac I Stelle des Polylinkers benachbart war. Hierdurch lag die cDNA in der falschen Orientierung vor und konnte daher nicht an die 5′-Hälfte der cDNA ligiert werden. Bei der in vitro Synthese der infektiösen RNA ist eine im Plasmid nur einmal auftretende Restriktionsschnittstelle nach dem polydA-Trakt (hier ungefähr 50 Nukleotide lang) hilfreich. Am besten ist hierzu die Asp 718 I am Ende des Polylinkers geeignet. Der Teil des Polylinkers, der zwischen der polydA-Sequenz und der Asp 718 I Stelle lag, wurde daher entfernt, indem das Plasmid 115 mit Eco RI geöffnet wurde, und die überhängenden Nukleotide mit "mung bean " Nuklease wegverdaut wurden. Durch Spaltung mit Asp 718 I wurde der verbleibende Teil des Polylinkers entfernt; die überhängenden Enden wurden mit dem Klenow Fragment der DNA Polymerase I aufgefüllt. Die modifizierten Enden wurden anschließend ligiert, wodurch Klon 115m erhalten wurde; diese Strategie regeneriert die Asp 718 Stelle, die nun dem PolydA Trakt benachbart liegt.

Die beiden Hälften der HRV2 cDNA wurden danach über die einzige Sph I Stelle vereinigt; das 1,2 kb Sph I/Asp 718 I Fragment des Klons 119 wurde durch das 3,2 kb Sph I/Asp 718 I Fragment des Klons 115m ersetzt; man erhielt den Klon pHRV2.

Bei der Transkription dieses Plasmids nach Linearisierung mit Asp 718 I entsteht eine RNA, die das ganze Genom von HRV2 umfaßt. Allerdings befinden sich am 5′-Ende 16 zusätzliche Nukleotide (welche der Sequenz von der Initiationsstelle der T7 RNA Polymerase bis zur ersten Base der cDNA entsprechen) und am 3′-Ende 5 zusätzliche Nukleotide von der Asp 718 I Stelle.

Die Transkription mit T7 RNA Polymerase wurde nach Standardmethoden ausgeführt (11). Das Transkriptionsgemisch wurde ohne weitere Behandlung direkt als RNA-Lösung verwendet. Die Qualität der RNA wurde auf einem 1.0% Agarosegel, das 0.1% SDS enthält, überprüft; üblicherweise waren mehr als 90% der RNA "full-length"-Moleküle. Die RNA-Konzentration wurde nach der Methode von Fleck and Begg (12) abgeschätzt. Die Nukleosidtriphosphate wurden durch zweimalige Ethanolfällung in Anwesenheit von 800 mM Ammoniumacetat entfernt, die Nukleinsäuren in Wasser gelöst, und die RNA wurde mit Alkali hydrolysiert. DNA und Proteine wurden mit Perchlorsäure ausgefällt. Mittels nachfolgender spektrophotometrischer Messung der Nukleotid-konzentration konnte unter der Annahme, daß 90% der RNA "full length"-Moleküle waren, die anfängliche RNA-Konzentration abgeschätzt werden.

Hela Zellen (Ohio Stamm) wurden unter Verwendung der DEAE-Dextranmethode transfiziert (9), wobei 300 µl der Transfektionsmischung mit RNA, von Asp 718 I geschnittenem pHRV2 transkribiert, pro Platte verwendet wurden. Nach 3 Tagen wurden die Zellen mit Neutralrot oder Kristallviolett gefärbt und auf die Anwesenheit von Plaques hin untersucht.
Überraschenderweise konnte keine Infektion beobachtet werden. Die mögliche Ursache hierfür könnten die zusätzlichen Nukleotide sein, obwohl Mizutani und Colonno (9) eine infektiöse RNA von einer 21 Nukleotide längeren cDNA von HRV14 erhalten hatten.
Um die Auswirkungen, die durch die Präsenz der zusätzlichen Nukleotide am 5′-Ende hervorgerufen werden, festzustellen, wurde eine alternative Strategie entwickelt, die die zusätzliche Sequenz auf nur zwei G-Reste reduziert. Eine völlige Eliminierung ist unmöglich, da die T7 RNA Polymerase zwei G Reste in den Positionen +1 und +2 benötigt. Van der Werf et al. (11) konnten zeigen, daß eine Stu I Stelle genau an der Initiationsstelle der Transkription der T7 Polymerase durch "site directed mutagenesis" eingeführt werden kann, indem die Sequenz AGGGCG (die Transkription startet mit dem ersten G) in AGGCCT geändert wird, wobei die beiden essentiellen G's für die Transkription mit der T7 RNA Polymerase unberührt bleiben. Das Restriktionsenzym Stu I schneidet nach dem zweiten G und erzeugt glatte Enden; daher enthält das RNA-Transkript eines Fragmentes, das in diese Stelle kloniert wurde, nur 2 zusätzliche G. Mit Hilfe eines "oligo-nucleotide directed mutagenesis kit" von Amersham wurde daher eine Stu I Stelle im Plasmid p100b eingeführt, wobei die Möglichkeit genutzt wurde, einzelsträngige DNA aus Bluescript Plasmiden zu erzeugen. Das resultierende Plasmid 100b Stu I wurde mit Stu I und Asp 718 I verdaut; das 7,0 kb Bam HI/Asp718 I Fragment von pHRV2 wurde dann mit Hilfe eines synthetischen, doppelsträngigen Oligonukleotides eingebaut, das die ersten neun Nukleotide der HRV2-Sequenz und entsprechende Enden enthielt, um korrekt in die Restriktionsstellen eingefügt werden zu können. Sequenzierung einiger der erhaltenen Kandidaten zeigte, daß alle mehr als eine Kopie des Oligonukleotides enthielten. Diese wurden durch Restriktionsverdau mit Bam HI, Eluierung des 10 kb Fragments von einem Agarosegel und Religation entfernt. Für einen Klon wurde gezeigt, daß er nur eine Kopie des Oligonukleotides enthielt; er wurde pHRV2/1 genannt.

Die Transkription und Transfektion wurde wie für pHRV2 beschrieben durchgeführt. Bei der Überprüfung der Zellen nach 3 Tagen wurden überraschenderweise Plaques mit einer Effizienz von 20-50 Plaques/µg erhalten. RNA aus Viruspartikeln erzeugte 100-200 Plaques/µg. Die Effizienz der in vitro transkribierten RNA entsprach also ungefähr der Hälfte derjenigen die mit viraler RNA erhalten wurde. Durch eine Kontrolltransfektion mit pHRV2/1 DNA konnte eindeutig nachgewiesen werden, daß die Infektion von RNA abhängig ist; die entsprechende DNA erzeugte nämlich keine Plaques.

Überraschenderweise, im Gegensatz zu den Ergebnissen von Mizutani und Colonno für HRV14 (9) war die Infektiösität der aus diesen Klonen transkribierten RNA jedoch abhängig von der Anzahl der zusätzlichen Nukleotide. Nur der "full size" Klon, der nicht mehr als die für die Transkription mit der T7 Polymerase notwendigen zusätzlichen zwei Guanine am 5′ Ende aufwies, ergab ein infektiöses Transkript.

Der durch die vorliegende Erfindung erstmals bereitgestellte, in vitro infektiöse RNA liefernde "full size" Klon pHRV2/1 bietet die wertvolle Gelegenheit, durch spezifische Mutagenese die strukturellen Erfordernisse für die Bindung an den "minor group receptor" zu untersuchen.

In diesem System lassen sich beliebige Mutationen untersuchen, die auf DNA-Ebene gezielt erzeugt werden können. Zur Untersuchung der Rezeptorbindungsstellen wurden in den viralen Hüllenproteinen von HRV2 Aminosäuren ausgetauscht, die folgende Kriterien erfüllen:
a. Geeignete AS-Reste müssen an der Oberfläche des Virions bzw. im "canyon", einem rund um die fünfzählige Symmetrieachse verlaufenden Einschnitt in der Virusoberfläche, liegen; dies läßt sich durch Vergleich mit der Kristallstruktur von HRV14 (5, 13) bestimmen.
b. Geeignete AS-Reste sollten innerhalb einer Rezeptorgruppe konserviert sein, was durch Sequenzvergleiche mit weiteren Serotypen (8) festgestellt werden kann.
c. Die nach (b) gefundenen AS-Reste können nur dann spezifisch für eine Rezeptorgruppe sein, wenn in der jeweils anderen Rezeptorgruppe an der entsprechenden Stelle der Sequenz davon verschiedene AS-Reste gefunden werden (Sequenzvergleiche mit Vertretern der "major receptor group" 4, 3, 7).

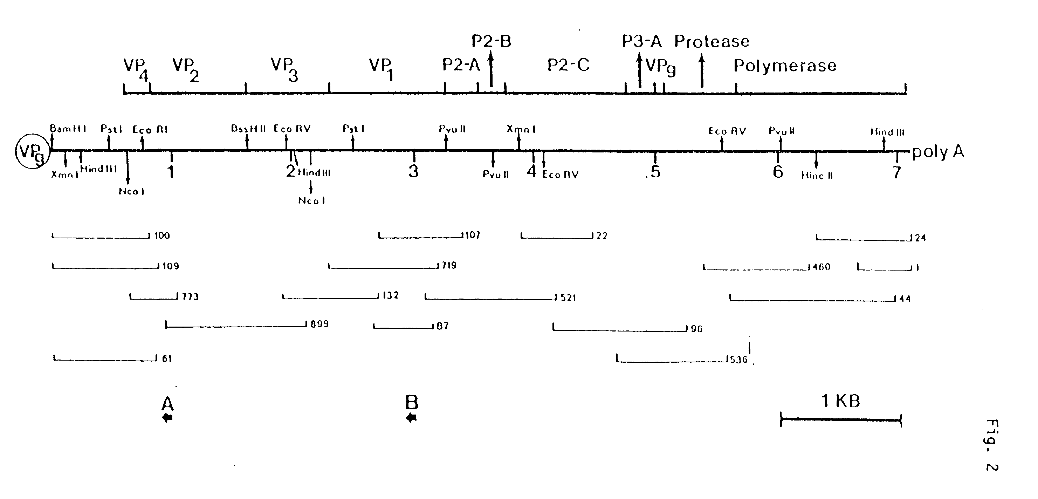

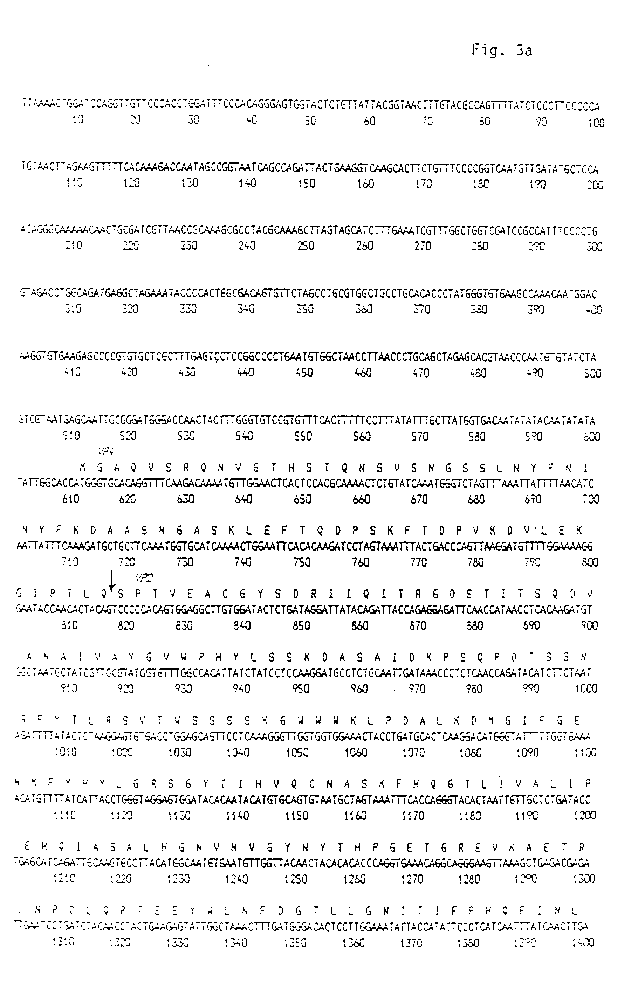

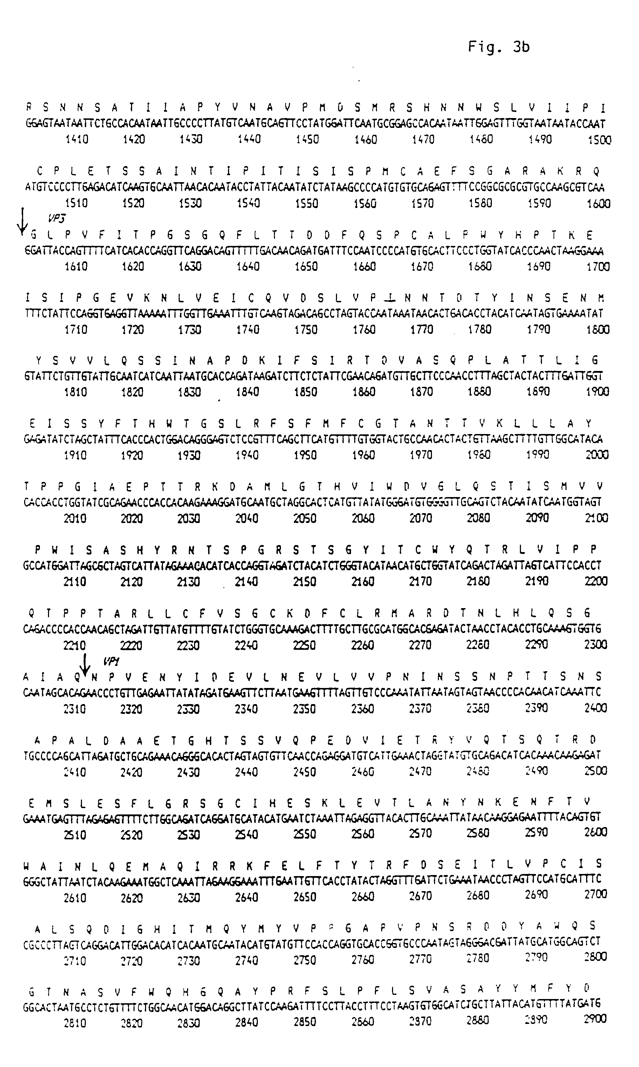

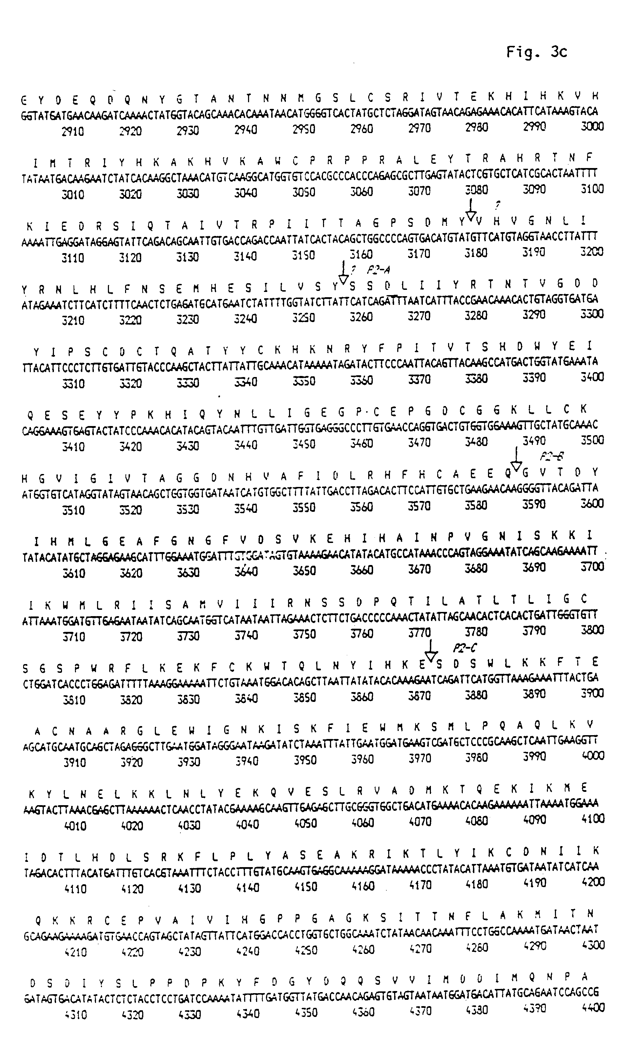

Gegenstand der vorliegenden Erfindung ist die Herstellung einer Mutante, bei der ein Arg (AS# 180) im VP3 von HRV2 gegen ein Thr ausgetauscht wurde. Dazu wurde wie folgt verfahren:
Um für die in vitro-Mutagenese ein Plasmid geeigneter Größe zu erhalten, wurde ein 0,4 kb großes Fragment der "full size" cDNA subkloniert (Abb. 4). Das Fragment umfaßt den Bereich von der HindIII-Schnittstelle (Nukleotid # 1982) bis zur PstI-Stelle (# 2422), und wurde in die PstI/HindIII-Stellen des Plasmids Bluescript (Stratagene, San Diego, California) eingebaut.

Entsprechend den Angaben von Stratagene wurde mittels eines Helferphagen die für die Mutagenese erforderliche, einzelsträngige DNA des Plasmids produziert und gereinigt. Die Mutagenese erfolgte mittels eines einzelsträngigen, synthetischen Oligonukleotids (26 Nukl.) mit einer vom Wildtyp abweichenden Sequenz an der gewünschten Stelle. Die Sequenz des Oligonukleotids lautet:
5′ - CCCAGATGTAGATGTACCTGGTGATG -3′
Mit Hilfe des "in vitro mutagenesis" Kits von Amersham wurde nach Vorschrift des Herstellers die gewünschte Mutante hergestellt und durch DNA-Sequenzierung nach Standardmethoden überprüft (14, 15). Da es das Vorhandensein weiterer PstI und HindIII-Stellen im Plasmid pHRV2/1 unmöglich machte, das die Mutation beinhaltende PstI-HindIII-Fragment direkt in den "full-size"-Klon zurückzuklonieren, wurde es vorerst in einem weiteren Subklon von pHRV2/1, der die Sequenz von der Eco RI-Stelle (# 743) bis zur ApaI-Stelle (# 3458) im Bluescript-Vektor enthält, über die PstI/HindIII-Schnittstelle einligiert (Abb. 4).

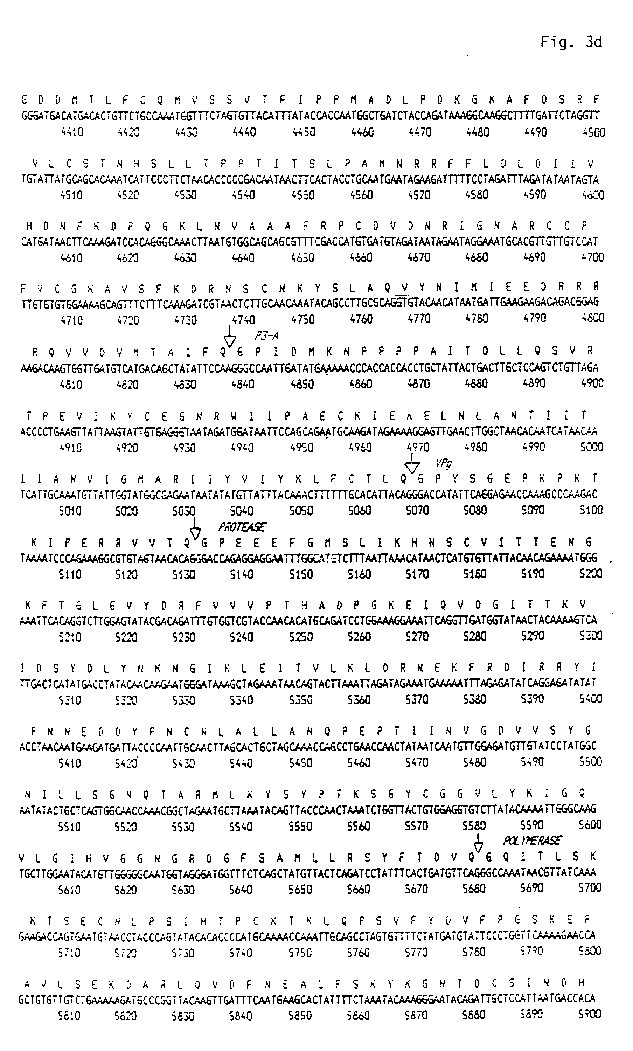

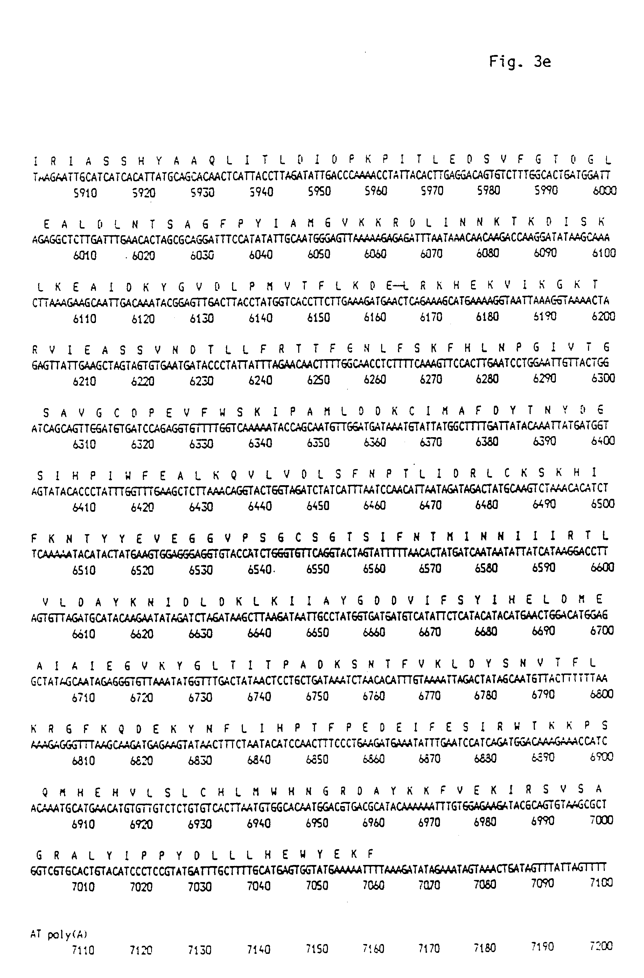

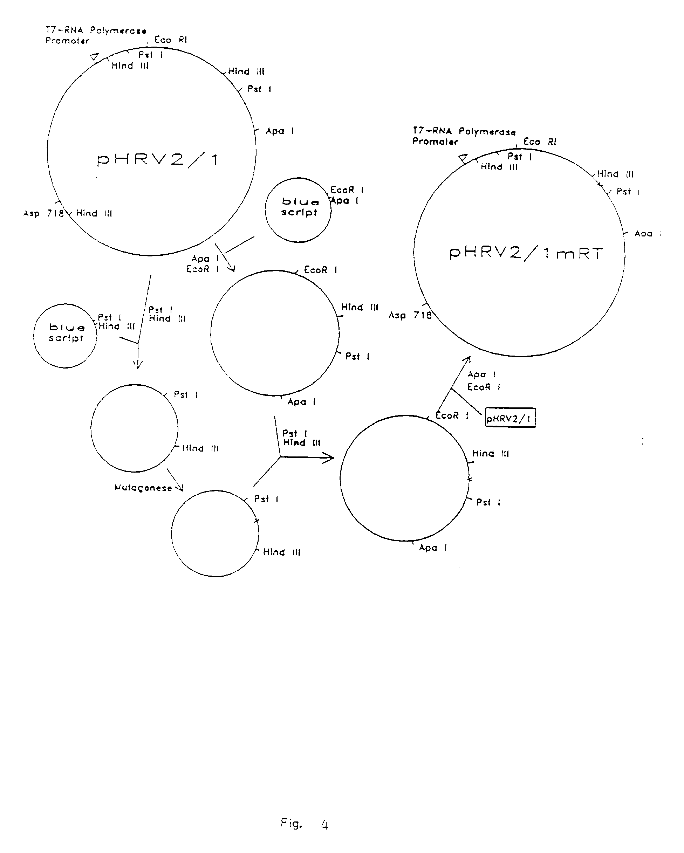

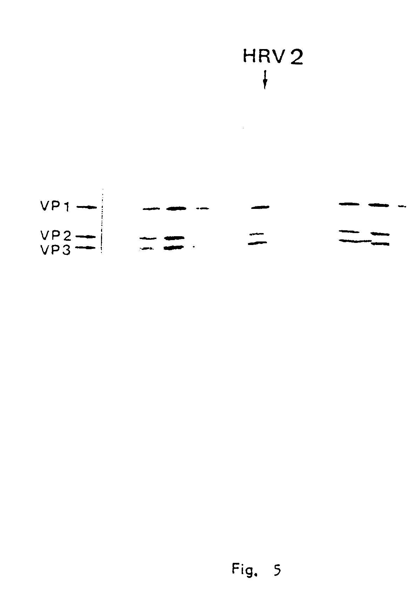

Das resultierende Plasmid wurde mit EcoRI und ApaI verdaut; das 2,7 kb große Fragment wurde dazu benutzt, die entsprechende Sequenz in pHRV2/1 zu ersetzen, wodurch pHRV2/mRT entstand. Die Transkription und Transfektion wurde, wie für pHRV2 beschrieben, durchgeführt. Aus einigen Plaques wurden Viren entnommen und HeLa-Zellmonolayer damit infiziert, wobei in drei Tagen 2x10⁷ - 2x10⁸ pfu/ml Medium gebildet wurden. Die entstandenen Viren wurden nochmals auf HeLa-Zellmonolayer vermehrt und durch drei Zentrifugationsschritte (10 min bei 500 x g; 30 min bei 20000 rpm, SW40; 60 min bei 45000 rpm, SW50-Rotor) gereinigt und pelletiert. Die in physiologischem Phosphatpuffer (PBS: 137 mM NaCl, 2,7 mM KCl, 8,1 mM KH₂PO₄, 1,5 mM Na₂HPO₄, pH 7,3) resuspendierten Viren wurden zur Herstellung ³⁵S-markierter Viruspartikel verwendet (16). Die Analyse auf einem denaturierenden Polyacrylamidgel (Abb. 5) ergab keinen Unterschied in der Wanderungsweite der einzelnen Virushüllproteine im Vergleich zum Wildtyp.

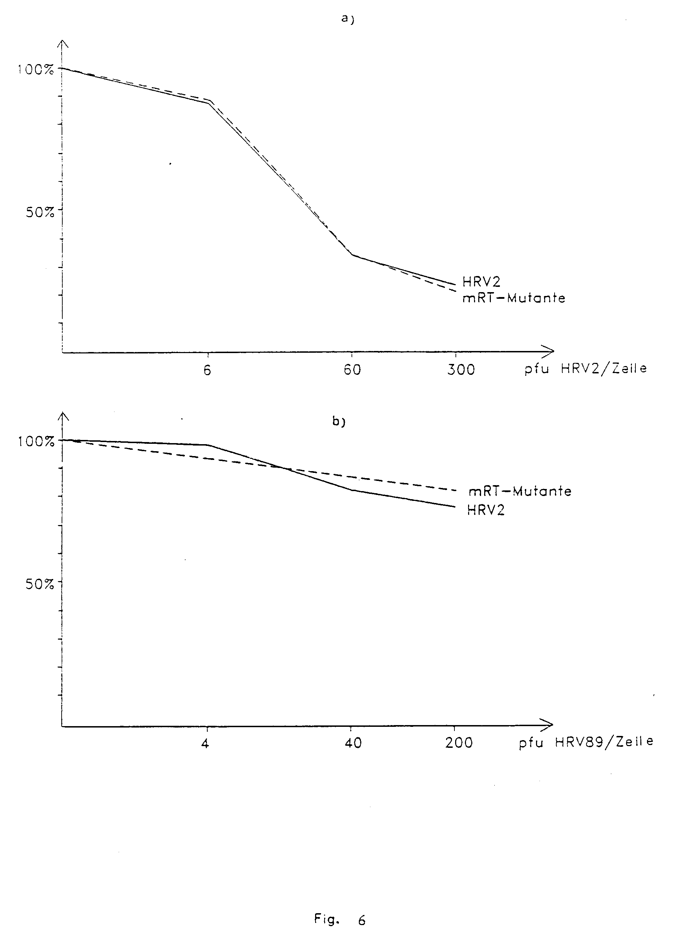

Um mögliche Änderungen des Bindungsverhaltens der Virusmutanten an die Wirtszelle feststellen zu können, wurden Bindungstests, wie von G. Abraham und R.J. Colonno (16) beschrieben, durchgeführt; die Inkubationsdauer betrug 16 Stunden für die Vorinkubation mit kaltem Virus bzw. vier Stunden für die Inkubation mit ³⁵S-markiertem Virus. Als kaltes Virus wurde HRV2 und HRV89 verwendet; verglichen wurden ³⁵S-markierter HRV2-Wildtyp und Mutante (Abb. 6). Es konnte kein signifikant abweichendes Bindungsverhalten der Virusmutante gegenüber dem des Wildtyps festgestellt werden (Abb. 6). Aus ca. 1x10⁸ gereinigten Viruspartikeln der Mutante wurde die RNA durch Phenolextraktion (17) isoliert und der die Mutation beinhaltende Bereich in cDNA transkribiert. Dafür wurde für die Erststrangsynthese ein synthetisches Oligonukleotid mit der Sequenz
5′ -CTTCTAATTTGAGCCATTTCTTG -3′
für die Zweitstrangsynthese das Oligonukleotid
5′ -TCTATTCCAGGTGAGGTT - 3′
verwendet. Die doppelsträngige cDNA wurde mit PstI und HindIII verdaut und in den Bluescript-Vektor einligiert. Die erhaltenen Plasmide wurden sequenziert. Auf diesem Weg konnte bestätigt werden, daß die Virusmutante tatsächlich die gewünschte Sequenzänderung beinhaltet.

Auf diese Weise lassen sich die Aminosäuren und die Regionen des Virions ermitteln, die für die Rezeptorbindung essentiell sind.

Aufgrund der bestehenden weit größeren Homologie des HRV2 zu den anderen, bisher sequenzierten Rhinoviren als HRV14 ist zu erwarten, daß diese Resultate mit höherer Sicherheit auch auf Rhinoviren Anwendung finden können, die zu der sogenannten "major receptor group" gehören.

Die vorliegende Erfindung stellt somit Plasmide bereit, die die vollständige c-DNA für HRV2 bzw. die durch spezifische Mutagenese erhältlichen Analoga unter der Kontrolle eines RNA Polymerase Promotors enthalten.

Gegenstand der vorliegenden Erfindung sind auch Systeme, die eine vollständige cDNA für HRV2 unter der Kontrolle eines RNA Polymerase Promotors enthalten, wobei die Kodons, die für Aminosäuren kodieren, die an der Oberfläche des Virions bzw. im "canyon" liegen, ausgetauscht sind gegen Kodons für andere Aminosäuren, insbesondere gegen Kodons für Aminosäuren, die das Bindungsverhalten gegenüber dem Rezeptor beeinflussen.

Bevorzugte Plasmide enthalten vor dem 1. Basenpaar der cDNA nur so viele Nukleotide, wie für die Initiation der Transkription erforderlich sind. Bei Verwendung der T7 RNA Polymerase sind dies beispielsweise die zwei essentiellen Guanine.

Von Vorteil ist es, in diesen Plasmiden zusätzlich eine nur einmal auftretende Restriktionsstelle im Anschluß an das 3′-Ende der cDNA vorzusehen.

Gegenstand der vorliegenden Erfindung ist selbstverständlich auch die von diesen Plasmiden durch Transkription erhältliche RNA.

Verwendet werden können die erfindungsgemäßen Plasmide beispielsweise zur Erzeugung viraler Polypeptide, indem geeignete Wirtsysteme mit diesen Plasmiden transformiert werden. Diese viralen Polypeptide können dann zur therapeutischen Behandlung, beispielsweise zur Stimulierung des Immunsystems und zur Bindung und/oder Blockierung der zellulären Rezeptoren für die Rhinoviren Verwendung finden.

### LEGENDE ZU DEN FIGUREN

### Figur 1

Schematische Darstellung des Zusammenbaus der vollständigen cDNA Kopie von HRV2, die unter der Kontrolle eines T7 RNA Polymerase Promotors steht. Die Fragmente, die in jedem Abschnitt zusammenligiert wurden, sind dick ausgezogen. Die Nummern über den Restriktionsstellen beziehen sich auf ihre Position auf der HRV2 Karte, außer jenen über den Sac I und Asp 718 I Stellen, die die Enden jedes cDNA Klons darstellen. Die folgenden Restriktionsstellen werden gezeigt: A, Asp 718 I; B,Bam HI; H, Hpa I; P, Pst I; R, Eco RI; S, Sac I; Se, Spe I; Sh, Sph I; Sm, Sma I. Alle Asp 718 I und Sac I Stellen und diejenigen Restriktionsstellen die mit einem Punkt markiert sind, stammen von Polylinkern der Vektors. Der T7 RNA Polymerase Promotor ist mit einem Pfeil markiert.

### Figur 2

Restriktionskarte des HRV2 Genoms.

### Figur 3

Sequenz des klonierten HRV2 Genoms und die daraus abgeleitete Aminosäuresequenz.

### Figur 4:

Schematische Darstellung der Subklonierung eines für die Mutagenese geeigneten Fragmentes, sowie seine Rückklonierung in den "full size" Klon von HRV2 nach der Mutagenese. Die mutierte Stelle ist durch ein "x" gekennzeichnet.

### Figur 5:

Autoradiogramm einer Gelelektrophorese. Auftrennung der viralen Hüllproteine aus verschiedenen Gradientenfraktionen einer Präparation von ³⁵S-markierten Viruspartikeln der Mutante im Vergleich zum Wildtyp.

### Figur 6:

Bindungstest der HRV2-Mutante. HeLa-Zellmonolayer wurden mit steigenden Konzentrationen an kaltem HRV2 (a) bzw. HRV89 (b) präinkubiert. Danach wurde die Mengen der ³⁵S-markierten Viruspartikel des Wildtyps bzw. der Mutante bestimmt, die noch gebunden werden konnten.

### BIBLIOGRAPHIE

1. Stott, E., J., and Killington, R.A., Ann.Rev.Microbiol., 26, 503-525 (1972).
2. Colonno, R. J., Callahan, P. L. and Long, W.J.,J. Virol. 57, 7-12 (1986).
3. Stanway,G., Hughes,P.J., Mountford,R.C., Minor,Ph.D., and Almond, J.W., Nucl. Acids Res., 12, 7859-7875 (1984).
4. Callahan,P.L., Mizutani,S. and Colonno,R.J.,Proc. Natl. Acad. Sci. U.S.A., 82, 732-736 (1985).
5. Rossmann,M.G., Arnold,E., Erickson,J.W., Frankenberger,E.A., Griffith,J.P., Hecht,H-J., Johnson,J.E., Kamer,G., Luo,M., Mosser,A.G., Rueckert,R.R., Sherry,B.A., and Vriend,G., Nature, 317, 145-154 (1985).
6. Skern,T., Sommergruber,W., Blaas,D., Gruendler,P., Frauendorfer,F., Pieler,C., Fogy,I., and Kuechler, E.,Nucl. Acids Res., 13, 2111-2126 (1985).
7. Duechler, M., Skern, T., Sommergruber, W., Neubauer, Ch., Gruendler, P., Fogy, I., Blaas, D. and Kuechler, E., Proc. Natl. Acad. Sci. U.S.A., 84, 2605-2609 (1987).
8. Hughes, P.J., North, C., Jellis, C.H., Minor, P.D., and Stanway, G., J. Gen. Virol. 69, 49-58 (1988).
9. Mizutani, S., and Colonno, R.,J.,J. Virol., 56, 628-632 (1985).
10. Skern, T., Sommergruber, W., Blaas, D., Pieler, C., and Kuechler, E.,Virology, 136, 125-132 (1984).
11. Van der Werf, S., Bradley, J., Wimmer, E., Studier, W., and Dunn, J.,J.,Proc. Natl. Acad. Sci. U.S.A., 83, 2330-2334 (1986).
12. Fleck, A. and Begg,D.,Biochim. Biophys. Acta, 108, 333 - 339 (1965).
13. Blaas, D., Küchler, E., Vriend, G., Arnold, E., Luo, M. and Rossmann, M.G., Proteins: Structure, Function and Genetics 2: 263-272 (1987)
14. Maxam, A. und Gilbert, W. Methods in Enzymology, Academic Press, New York 65: 499 - 560 (1980).
15. Chen, E.Y. and Seeburg, P.H. DNA 4: 165 - 179 (1985).
16. Abraham, G. and Colonno, R.J., J. Virol. 51, 340-345 (1984).
17. Lee, Y.F., Kitamura, N. Nomoto, A. and Wimmer, E.J. Gen. Virol. 44, 311-322 (1979).
18. Racaniello, V.R., Baltimore, D. Science 214, 916-919 (1981).

## Patentansprüche (Patentansprüche für folgende(n) Vertragsstaat(en): AT, BE, CH, LI, DE, FR, GB, IT, LU, NL, SE)

1. Plasmid, dadurch gekennzeichnet, daß es
a) die vollständige cDNA für HRV2 oder die durch spezifische Mutagenese erhältlichen Analoga unter der Kontrolle des T7 RNA Polymerase Promotors enthält, wobei
b) vor dem ersten Basenpaar der cDNA zwei für die T7 RNA Polymerase essentiellen Guanine enthalten sind.

2. Plasmid gemäß Anspruch 1, dadurch gekennzeichnet, daß es die vollständige cDNA für HRV2 unter der Kontrolle eines RNA Polymerase Promotors enthält, wobei die Kodons, die für Aminosäuren kodieren, die an der Oberfläche des Virions bzw. im "canyon" liegen, ausgetauscht sind gegen Kodons für andere Aminosäuren, insbesondere gegen Kodons für Aminosäuren, die das Bindungsverhalten gegenüber dem Rezeptor beeinflussen.

3. Plasmid nach einem der vorhergehenden Ansprüche, dadurch gekennzeichnet, daß es zusätzlich eine nur einmal auftretende Restriktionsstelle im Anschluß an das 3'-Ende der cDNA aufweist

4. RNA, herstellbar durch Transkription von einem der Plasmide gemäß Anspruch 1 bis 3.

## Patentansprüche (Patentansprüche für folgende(n) Vertragsstaat(en): ES, GR)

1. Plasmid, dadurch gekennzeichnet, daß es
a) die vollständige cDNA für HRV2 oder die durch spezifische Mutagenese erhältlichen Analoga unter der Kontrolle des T7 RNA Polymerase Promotors enthält, wobei
b) vor dem ersten Basenpaar der cDNA zwei für die T7 RNA Polymerase essentiellen Guanine enthalten sind.

2. Plasmid gemäß Anspruch 1, dadurch gekennzeichnet, daß es die vollständige cDNA für HRV2 unter der Kontrolle eines RNA Polymerase Promotors enthält, wobei die Kodons, die für Aminosäuren kodieren, die an der Oberfläche des Virions bzw. im "canyon" liegen, ausgetauscht sind gegen Kodons für andere Aminosäuren, insbesondere gegen Kodons für Aminosäuren, die das Bindungsverhalten gegenüber dem Rezeptor beeinflussen.

3. Plasmid nach einem der vorhergehenden Ansprüche, dadurch gekennzeichnet, daß es zusätzlich eine nur einmal auftretende Restriktionsstelle im Anschluß an das 3'-Ende der cDNA aufweist

4. RNA, herstellbar durch Transkription von einem der Plasmide gemäß Anspruch 1 bis 3.

5. Verfahren zur Herstellung von RNA, dadurch gekennzeichnet, daß die RNA durch Transkription von einem der Plasmide gemäß Anspruch 1 bis 3 hergestellt wird.

## Claims (Claims for the following Contracting State(s): AT, BE, CH, LI, DE, GB, IT, LU, NL, SE)

1. Plasmid, characterised in that
a) it contains the complete cDNA for HRV2 or the analogs obtainable by specific mutagenesis under the control of the T7 RNA polymerase promoter,
b) two guanines which are essential for the T7 RNA polymerase being contained before the first base pair of the cDNA.

2. Plasmid according to claim 1, characterised in that it contains the complete cDNA for HRV2 under the control of an RNA polymerase promoter, the codons which code for amino acids which are located on the surface of the virion or in the "canyon" being exchanged for codons for other amino acids, particularly codons for amino acids which influence the binding characteristics relative to the receptor.

3. Plasmid according to one of the preceding claims, characterised in that it additionally contains, adjoining the 3'-end of the cDNA, a restriction site which occurs only once.

4. RNA which may be prepared by transcribing one of the plasmids according to claims 1 to 3.

## Claims (Claims for the following Contracting State(s): ES, GR)

1. Plasmid, characterised in that
a) it contains the complete cDNA for HRV2 or the analogs obtainable by specific mutagenesis under the control of the T7 RNA polymerase promoter,
b) two guanines which are essential for the T7 RNA polymerase being contained before the first base pair of the cDNA.

2. Plasmid according to claim 1, characterised in that it contains the complete cDNA for HRV2 under the control of an RNA polymerase promoter, the codons which code for amino acids which are located on the surface of the virion or in the "canyon" being exchanged for codons for other amino acids, particularly codons for amino acids which influence the binding characteristics relative to the receptor.

3. Plasmid according to one of the preceding claims, characterised in that it additionally contains, adjoining the 3'-end of the cDNA, a restriction site which occurs only once.

4. RNA which may be prepared by transcribing one of the plasmids according to claims 1 to 3.

5. Process for preparing RNA, characterised in that the RNA is prepared by transcription of one of the plasmids according to claims 1 to 3.

## Revendications (Revendications pour l'(les) Etat(s) contractant(s) suivant(s): AT, BE, CH, LI, DE, FR, GB, IT, LU, NL, SE)

1. Plasmide caractérisé en ce que:
a) il contient l'ADNc complet pour HRV2 ou les analogues qui peuvent être obtenus par mutagenèse spécifique sous le contrôle du promoteur de l'ARN polymérase de T7,
b) deux guanines essentielles pour l'ARN polymérase de T7 sont contenues en amont de la première paire de bases de l'ADNc.

2. Plasmide selon la revendication 1, caractérisé en ce qu'il contient l'ADNc complet pour HRV2 sous le contrôle d'un promoteur d'ARN polymérase, les codons qui codent des acides aminés qui sont situés à la surface du virion ou dans le "canyon" étant remplacés par des codons pour d'autres acides aminés, en particulier par des codons pour des acides aminés qui influent sur le comportement de liaison vis à vis du récepteur.

3. Plasmide selon l'une des revendications précédentes, caractérisé en ce qu'il présente en outre à la suite de l'extrémité 3' de l'ADNc un site de restriction qui n'apparaît qu'une fois.

4. ARN qui peut être préparé par transcription de l'un des plasmides selon les revendications 1 à 3.

## Revendications (Revendications pour l'(les) Etat(s) contractant(s) suivant(s): ES, GR)

1. Plasmide caractérisé en ce que:
a) il contient l'ADNc complet pour HRV2 ou les analogues qui peuvent être obtenus par mutagenèse spécifique sous le contrôle du promoteur de l'ARN polymérase de T7,
b) deux guanines essentielles pour l'ARN polymérase de T7 sont contenues en amont de la première paire de bases de l'ADNc.

2. Plasmide selon la revendication 1, caractérisé en ce qu'il contient l'ADNc complet pour HRV2 sous le contrôle d'un promoteur d'ARN polymérase, les codons qui codent des acides aminés qui sont situés à la surface du virion ou dans le "canyon" étant remplacés par des codons pour d'autres acides aminés, en particulier par des codons pour des acides aminés qui influent sur le comportement de liaison vis à vis du récepteur.

3. Plasmide selon l'une des revendications précédentes, caractérisé en ce qu'il présente en outre à la suite de l'extrémité 3' de l'ADNc un site de restriction qui n'apparaît qu'une fois.

4. ARN qui peut être préparé par transcription de l'un des plasmides selon les revendications 1 à 3.

5. Procédé de préparation d'ARN, caractérisé en ce que l'ARN est préparé par transcription de l'un des plasmides selon les revendications 1 à 3.
